(19) Europäisches Patentamt
European Patent Office
Office européen des brevets

(11) Veröffentlichungsnummer : **0 573 392 A1**

(12) # EUROPÄISCHE PATENTANMELDUNG

(21) Anmeldenummer : **93810382.7**

(22) Anmeldetag : **25.05.93**

(51) Int. Cl.[5] : **C07D 277/54,** A61K 31/425, C07D 417/12

(30) Priorität : **03.06.92 CH 1778/92**
**03.06.92 CH 1779/92**
**06.10.92 CH 3114/92**

(43) Veröffentlichungstag der Anmeldung :
**08.12.93 Patentblatt 93/49**

(84) Benannte Vertragsstaaten :
**AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Missbach, Martin, Dr.**
**Cristalinweg 4**
**CH-4310 Rheinfelden (CH)**

(54) **Thiosemicarbazonderivate und ihre Anwendung als Arzneimittelwirkstoff.**

(57) Die Erfindung betrifft neue Thiosemicarbazone der Formel I

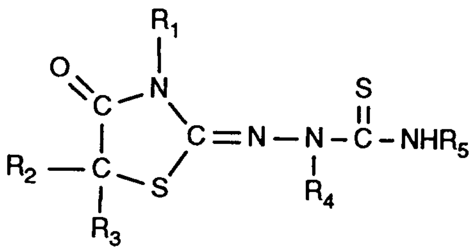

(I)

worin $R_1$ Niederalkyl, Niederalk-2-en-1-yl oder auch Niederalk-2-in-1-yl bedeutet,
$R_2$ und $R_3$ unabhängig voneinander Wasserstoff oder Niederalkyl bedeuten oder gemeinsam für Niederalkyliden stehen,
$R_4$ Wasserstoff, Niederalkyl, Niederalkoxy, Aryl oder Arylniederalkyl oder die Gruppe -C(=O)-$R_6$ bedeutet, in der $R_6$ für Niederalkyl, Aryl, Heteroaryl, Aryloxy, Arylniederalkoxy oder Nieder-2-en-1-yloxy steht,
$R_6$ Aryl, Arylniederalkyl, ungesättigtes oder gesättigtes Heterocyclylniederalkyl, Niederalkoxycarbonylniederalkyl oder die Gruppe -C(=O)-$R_7$ bedeutet, in der $R_7$ für Niederalkyl, Aryl, Arylniederalkyl, Arylniederalkenyl, Heteroaryl, Aryloxy, Arylniederalkoxy oder Nieder-2-en-1-yloxy steht oder $R_5$ auch Niederalkyl, Niederalk-2-en-1-yl oder Niederalk-2-in-1-yl bedeutet, wenn $R_4$ verschieden von Wasserstoff, und ihre Salze, Verfahren zur Herstellung der genannten Verbindungen, diese enthaltende pharmazeutische Präparate und ihre Verwendung als Arzneimittelwirkstoff.

EP 0 573 392 A1

Die Erfindung betrifft neue Thiosemicarbazone der Formel I

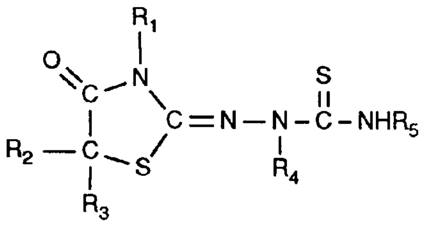

(I)

worin $R_1$ Niederalkyl, Niederalk-2-en-1-yl oder auch Niederalk-2-in-1-yl bedeutet, $R_2$ und $R_3$ unabhängig voneinander Wasserstoff oder Niederalkyl bedeuten oder gemeinsam für Niederalkyliden stehen,
$R_4$ Wasserstoff, Niederalkyl, Niederalkoxy, Aryl, Arylniederalkyl oder die Gruppe -C(=O)-$R_6$ bedeutet, in der $R_6$ für Niederalkyl, Aryl, Heteroaryl, Aryloxy, Arylniederalkoxy oder Niederalk-2-en-1-yloxy steht,
$R_6$ Aryl, Arylniederalkyl, ungesättigtes oder gesättigtes Heterocyclylniederalkyl, Niederalkoxycarbonylniederalkyl oder die Gruppe -C(=O)-$R_7$ bedeutet, in der $R_7$ für Niederalkyl, Aryl, Arylniederalkyl, Arylniederalkenyl, Heteroaryl, Aryloxy, Arylniederalkoxy oder Niederalk-2-en-1-yloxy steht oder $R_6$ auch Niederalkyl, Mederalk-2-en-1-yl oder Niederalk-2-in-1-yl bedeutet, wenn $R_4$ verschieden von Wasserstoff ist, und ihre Salze, Verfahren zur Herstellung der genannten Verbindungen, diese enthaltende pharmazeutische Präparate und ihre Verwendung als Arzneimittelwirkstoff.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalk-2-en-1-yl ist beispielsweise $C_3$-$C_5$-Alk-2-en-1-yl, wie insbesondere Allyl oder Methallyl.

Niederalk-2-in-1-yl ist beispielsweise $C_3$-$C_5$-Alk-2-in-1-yl, wie insbesondere Prop-2-in-1-yl oder auch But-2-in-1-yl.

Niederalkyl ist beispielsweise $C_1$-$C_4$-Alkyl, wie Methyl, Ethyl, Propyl oder Butyl.

Niederalkyliden ist beispielsweise $C_1$-$C_4$-Alkyliden, wie insbesondere Methylen oder auch Ethyliden.

Niederalkoxy ist z.B. n-Propoxy, Isopropoxy, n-Butoxy oder tert.-Butoxy, vorzugsweise Ethoxy und vor allem Methoxy.

Aryl - als solches und auch in zusammengesetzten Begriffen wie z.B. Arylniederalkyl ist z.B. Phenyl oder Naphthyl, wie 1- oder 2-Naphthyl, oder substituiertes Phenyl oder Naphthyl, wie z.B. Phenyl oder Naphthyl, welche durch Niederalkyl,
Hydroxy-niederalkyl, Halogen-niederalkyl, Hydroxy, Niederalkoxy, Niederalkanoyloxy, Halogen, Cyano und/oder Nitro substituiert sind. Aryl ist bevorzugt unsubstituiertes oder wie oben angegeben substituiertes Phenyl, und insbesondere Phenyl.

Arylniederalkyl ist vorzugsweise Phenylniederalkyl und insbesondere Benzyl.

Arylniederalkenyl ist vorzugsweise Phenylniederalkenyl, insbesondere Phenylallyl oder auch Phenylvinyl (Phenylethylen).

Aryloxy ist beispielsweise Phenyloxy.

Arylniederalkoxy ist z.B. Phenyl-$C_1$-$C_4$-alkoxy, insbesondere Benzyloxy.

Niederalk-2-en-1-yloxy ist beispielsweise $C_3$-$C_5$-Alk-2-en-1-yloxy, wie insbesondere Allyl- oder Methallyloxy.

Niederalkoxycarbonylniederalkyl ist z.B. Methoxy- oder Ethoxycarbonylmethyl oder auch -ethyl.

Ungesättigstes Heterocyclylniederalkyl ist z.B.Hetarylniederalkyl.
Unter Hetaryl in zusammengesetzten Begriffen wie z.B. Hetarylniederalkyl - ist ein insbesondere monocyclischer, aber auch bi- oder polycyclischer, heterocyclischer Rest aromatischen Charakters zu verstehen. Bi- und polycyclisches Hetaryl kann aus mehreren heterocyclischen Ringen zusammengesetzt sein oder bevorzugt aus einem Heterocyclus und einem oder mehreren, z.B. einem oder zwei und insbesondere einem, annellierten carbocyclischen Ring, insbesondere Benzoring, bestehen. Jeder einzelne Ring enthält z.B. 3, 5, 6 oder 7 und insbesondere 5 oder 6 Ringglieder. Hetaryl steht insbesondere für einen aza-, thia-, oxa-, thiaza-, thiadiaza-, oxaza-, diaza-, triaza- und tetrazacyclischen Rest.

Hetaryl steht in erster Linie für monocyclische monoaza-, monothia- oder monooxacyclische Reste, wie etwa Pyrryl, z.B. 2-Pyrryl oder 3-Pyrryl, Pyridyl, z.B. 2-, 3- oder 4-Pyridyl, Thienyl, z.B. 2- oder 3-Thienyl, oder Furyl, z.B. 2-Furyl; bicyclische monoaza-, monooxa- oder monothiacyclische Reste, wie Indolyl, z.B. 2- oder 3-Indolyl, Chinolinyl, z.B. 2- oder 4-Chinolinyl, Isochinolinyl, z.B. 1-Isochinolinyl, Benzofuranyl, z.B. 2- oder 3-Benzofuranyl, oder Benzothienyl, z.B. 2- oder 3-Benzothienyl; monocyclische diaza-, triaza-, tetraza, oxaza-,

thiaza- oder thiadiazacyclische Reste, wie Imidazolyl, z.B. 2-Imidazolyl, Pyrimidinyl, z.B. 2- oder 4-Pyrimidinyl, Triazolyl, z.B. 1,2,4-Triazol-3-yl, Tetrazolyl, z.B. 1- oder 5-Tetrazolyl, Oxazolyl, z.B. 2-Oxazolyl, Isoxazolyl, z.B. 3- oder 4-Isoxazolyl, Thiazolyl, z.B. 2-Thiazolyl, Isothiazolyl, z.B. 3- oder 4-Isothiazolyl oder 1,2,4- oder 1,3,4-Thiadiazolyl, z.B. 1,2,4-Thiadiazol-3-yl oder 1,3,4-Thiadiazol-2-yl; oder bicyclische diaza-, oxaza- oder thiazacyclische Reste, wie Benzimidazolyl, z.B. 2-Benzimidazolyl, Benzoxazolyl, z.B. 2-Benzoxazolyl, oder Benzthiazolyl, z.B. 2-Benzthiazolyl.

Hetarylreste sind unsubstituiert oder tragen Substituenten. Als Substituenten an Ringkohlenstoffatomen kommen z.B. die oben für Arylreste angegebenen Substituenten in Frage und zusätzlich Oxo (=O). Ringstickstoffatome können z.B. durch Niederalkyl, Arylniederalkyl, Niederalkanoyl, Benzoyl, Carboxy, Niederalkoxycarbonyl, Hydroxy, Niederalkoxy, Niederalkanoyloxy oder Oxido ($-\overline{O}|$) substituiert sein.

Hetaryl ist vor allen Dingen Pyridyl, Thienyl, Pyrryl oder Furyl.

Hetarylniederalkyl ist vor allen Dingen Pyridyl-, Thienyl-, Pyrryl- oder Furylmethyl. Gesättigtes Heterocyclylniederalkyl enthält einen 5- oder 6- gliedrigen gesättigten heterocyclischen Ring, der ein Stickstoff- oder Sauerstofatom aufweist und steht insbesondere für einen aza- oder oxacyclischen Rest, der gegebenfalls substituiert sein kann.
Ein gesättigter 6-gliedriger heterocyclischer Ring kann neben einem Sauerstoffatom zusätzlich noch ein Stickstoffatom enthalten.

Ein gesättigter 5- oder 6-gliedriger heterocyclischer Ring steht beispielsweise für Pyrrolidinyl, Piperidino, Piperidyl, Tetrahydrofuranyl oder Tetrahydropyranyl, worin ein oder mehrere Wasserstoffatome durch einen oder mehrere Substituenten, beispielsweise durch Niederalkyl ersetzt sein können.
Ein gesättigter 6-gliedriger heterocyclischer Rest, der neben einem Sauerstoffatom auch ein Stickstoffatom enthält ist beispielsweise Morpholino oder auch Morpholinyl. Gesättigtes Heterocyclylniederalkyl ist vor allen Dingen Pyrrolidinyl-, Tetrahydrofuranyl- oder auch Tetrahydropyranylmethyl.

Pharmazeutisch verwendbare Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren oder gegebenenfalls ungesättigten oder hydroxylierten aliphatischen Dicarbonsäuren, z.B. Acetate, Oxalate, Malonate, Maleinate, Fumarate, Maleate, Tartrate oder Citronate. Salze von Verbindungen der Formel I sind beispielsweise deren Säureadditionssalze, beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Die Verbindungen der Formel I und ihre pharmazeutisch verwendbaren Salze weisen wertvolle pharmakologische Eigenschaften auf. Insbesondere weisen sie ausgeprägte antiarthritische Eigenschaften auf. Diese können in vivo beispielsweise am Modell der Adjuvans-Arthritis der Ratte nach I. Wiesenberg et al. Clin. Exp. Immunol. 78, 245 (1989) im Dosisbereich ab etwa 0,1 bis etwa 10,0 mg/kg p.o. oder i.p., insbesondere ab etwa 0,1 bis etwa 3,0 mg/kg p.o. oder i.p. nachgewiesen werden.

Die Verbindungen der Formel I und ihre pharmazeutisch verwendbaren Salze können deshalb zur Behandlung von Erkrankungen des rheumatoiden Formenkreises eingesetzt werden. Zu diesen gehören insbesondere die rheumatoide Arthritis, die juvenile Arthritis, die ankylosierende Spondylitis und andere seronegative Spondylathritiden, z.B. Spondylarthritis bei Colitis ulcerosa und Morbus Crohn, aber auch reaktive Arthritiden, Kollagenkrankheiten wie Lupus erythematosus, degenerative rheumatische Erkrankungen, extraartikuläre rheumatische und pararheumatische Erkrankungen, z.B. Gicht und Osteoporose.

Die Erfindung betrifft in erster Linie Verbindungen der Formel I, worin $R_1$ $C_1$-$C_4$-Alkyl, $C_3$-$C_5$-Alk-2-en-1-yl oder $C_3$-$C_5$-Alk-2-in-1-yl bedeutet,
$R_2$ und $R_3$ unabhängig voneinander Wasserstoff, gleiche $C_1$-$C_4$-Alkyl oder $C_1$-$C_4$-Alkyliden darstellen und $R_4$ Wasserstoff, $C_1$-$C_4$-Alkyl, $C_1$-$C_2$-Alkoxy, Phenyl oder Phenylniederalkyl oder die Gruppe -C(=O)-$R_6$ bedeutet, in der $R_6$ für $C_1$-$C_4$-Alkyl, Phenyl, Naphthyl, Pyridyl, Thienyl, Pyrryl, Furyl, Phenyloxy, Phenyl-$C_1$-$C_4$-alkoxy oder $C_3$-$C_5$-Alk-2-en-1-yloxy steht,
$R_6$ Phenyl, Naphthyl, Phenylniederalkyl, Pyridyl-, Thienyl-, Pyrryl- oder Furyl-, Pyrrolidinyl-, Tetrahydrofuranyl- oder Tetrahydropyranylniederalkyl oder $C_1$-$C_4$-Alkoxy-carbonyl-C1-C4-alkyl oder die Gruppe -C(=O)-$R_7$bedeutet, in der $R_7$ die für $R_4$ angegebenen Bedeutungen von $R_6$ hat oder Phenylniederalkyl oder auch Phenylniederalkenyl bedeutet oder $R_5$ auch $C_1$-$C_4$-Alkyl, $C_3$-$C_5$-Alk-2-en-1yl oder $C_3$-$C_5$-Alk-2-in-1yl bedeutet, wenn $R_4$ verschieden von Wasserstoff ist, und ihre Salze, insbesondere pharmazeutisch verwendbaren Salze.

Die Erfindung betrifft vor allem Verbindungen der Formel I, worin $R_1$ $C_3$-$C_5$-Alk-2-en-1-yl, wie Allyl oder Methallyl oder $C_3$-$C_5$-Alk-2-in-1-yl, wie Prop-2-in-1-yl bedeutet. $R_2$ und $R_3$ beide Wasserstoff oder gleiche $C_1$-$C_4$-Alkyl, wie Methylgruppen bedeuten, $R_4$ Wasserstoff, $C_1$-$C_4$-Alkyl, wie Methyl oder Ethyl, $C_1$-$C_2$-Alkoxy, wie Methoxy oder Ethoxy, Phenyl, Phenylniederalkyl, wie z.B. Benzyl oder Phenylethyl oder die Gruppe -C(=O)-$R_6$bedeutet, in der $R_6$ für $C_1$-$C_4$-Alkylgruppen, wie z.B. die Methylgruppe, Phenyl, Pyridyl, Thienyl, Phenyloxy, Benzyloxy oder $C_3$-$C_5$-Alk-2-en-1-yloxy, wie Allyl- oder Methallyloxy steht,
und $R_6$ Phenyl, Phenylniederalkyl, wie z.B. Benzyl oder Phenylethyl, Pyridyl-, Thienyl-, Pyrryl-, Furyl- oder Pyrrolidinyl-, Tetrahydrofuranyl- oder Tetrahydropyranylniederalkyl, wie z.B. Pyridyl-, Thienyl-, Pyrryl-, oder Furyl- Pyrrolidinyl-, Tetrahydrofuranyl- oder Tetrahydropyranylmethyl, $C_1$-$C_4$-Alkoxycarbonyl-$C_1$-$C_4$-alkyl, wie z.B. Methoxy- oder ethoxycarbonylmethyl oder -ethyl oder die Gruppe -C(=O)-$R_7$ bedeutet, in der $R_7$ die für $R_4$ angegebenen Bedeutungen von $R_6$ hat oder auch Benzyl oder Phenylallyl bedeutet oder $R_5$ auch $C_1$-$C_4$-Alykl, wie z.B. Methyl, $C_3$-$C_5$-Alk-2-en--1-yl, wie z.B. Allyl oder Methallyl, $C_3$-$C_5$-Alk-2-in-1-yl, wie z.B. Prop-2-in-1yl bedeutet, wenn $R_4$ verschieden von Wasserstoff ist, und ihre Salze, insbesondere pharmazeutisch verwendbare Salze.

Die Erfindung betrifft insbesondere Verbindungen der Formel I, worin $R_1$ Allyl oder Methallyl, $R_2$ und $R_3$ beide Wasserstoff und $R_4$ Wasserstoff, Methyl oder Methoxy oder die Gruppe -C(=O)-$R_6$ bedeuten, in der $R_6$ für Methyl, Phenyl, Phenyloxy, Benzyloxy oder Allyloxy steht und $R_5$ Phenyl, Benzyl, Pyridyl-, Furyl- oder Tetrahydrofuranylmethyl oder Methoxycarbonylmethyl oder die Gruppe -C(=O)-$R_7$ bedeutet, in der $R_7$ die für $R_4$ angegebenen Bedeutungen von $R_6$ hat oder auch Benzyl oder Phenylallyl bedeutet oder $R_6$ auch Methyl, Allyl, Methallyl, Prop-2-in-1y1 bedeutet, wenn $R_4$ verschieden von Wasserstoff ist, ihre Salze, insbesondere pharmazeutisch verwendbaren Salze.

Die Erfindung betrifft namentlich die in den Beispielen genannten Verbindungen der Formel I und ihre Salze, insbesondere ihre pharmazeutisch verwendbaren Salze.

Verbindungen der Formel I können in an sich bekannter Weise hergestellt werden, indem man a) eine Verbindung der Formel II

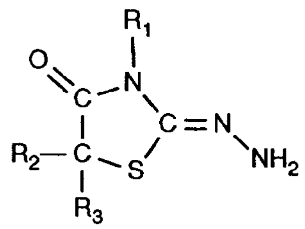

(II)

in der $R_1$, $R_2$ und $R_3$ die oben angegebenen Bedeutungen haben, mit einer Isothiocyanatverbindung der Formel III

$R_6$-NCS (III)

umsetzt.

Die Kondensation der Verbindungen der Formel II mit Verbindungen der Formel III erfolgt in üblicher Weise in einem protischen oder aprotischen Lösungsmittel, wie z.B. eines aliphatischen Halogenkohlenwasserstoffes, wie in Dichlormethan, insbesondere Methylenchlorid, oder eines aliphatischen oder cycloaliphatischen Ethers, z.B. in Tetrahydrofuran oder auch Dioxan. Ferner kommen als Lösungsmitel beispielsweise noch Acetonitril, Ethanol und Toluol in Frage.

Die Substanzen werden bei einer Temperatur von -50 - +120°C, vorteilhafterweise bei 0 - 80°C gegebenenfalls in Gegenwart eines basischen Kondensationsmittels wie z.B. Dimethylaminopyridin, Triethylamin, Chinolin oder Pyridin umgesetzt.

Ausgangsverbindungen der obigen Formel III, wenn $R_5$ die Gruppe -C(=O)-$R_7$ bedeutet, sind zum Teil instabil und werden gegebenfalls in status nascendi aus entsprechenden Säurehalogeniden, insbesondere Säurechloride der Formel IIIa

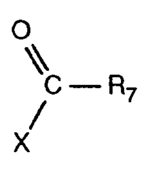

(IIIa)

worin $R_7$ die oben angegebenen Bedeutungen hat und X ein Halogenatom, insbesondere Chloratom bedeutet,

durch Umsetzung mit einem Metallthioisocyanat, beispielsweise Alkalimetallthioisocyanat oder Ammoniumthioisocyanat erhalten.

Die Ausgangsverbindungen der Formel II sind bekannt und in der DE-OS-2,035,419 beziehungsweise in der schweizerischen Patentschrift Nr. 511,877 beschrieben.

Die Isothiocyanatverbindungen der Formel III können im allgemeinen jedoch aus den entsprechenden Aminen der Formel IV

$$R_5\text{-}NH_2 \qquad (IV)$$

in der $R_5$ die oben angegebene Bedeutung hat durch Behandlung mit Thiophosgen hergestellt werden.

Gemäss einer weiteren Verfahrensvariante b) zu Verfahren a) können jedoch auch Verbindungen der Formel I hergestellt werden, indem man Verbindungen der Formel II mit einer Verbindung der Formel V

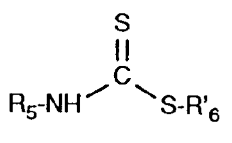

(V)

in der $R_5$ die oben angegebene Bedeutung hat und $R'_6$ Niederalkyl bedeutet, unter analogen Kondensationsbedingungen, wie oben unter Verfahrensvariante a) beschrieben, umsetzt.

Verbindungen der Formel V werden erhalten, indem man die entsprechenden Amine der Formel IV $R_5NH_2$ mit Schwefelkohlenstoff umsetzt und nachträglich mit einem Niederalkylhalogenid, insbesondere Niederalkyljodid behandelt.

Nach einem weiteren Verfahren c) lassen sich Verbindungen der Formel I herstellen, indem man Verbindungen der Formel VI

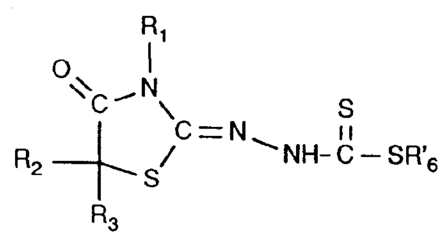

(VI)

in der $R_1$, $R_2$ und $R_3$ die oben angegebenen Bedeutungen haben und $R'_6$ einen Niederalkylrest bedeutet, mit entsprechenden Aminen der oben angegebenen Formel IV

$$R_5\text{-}NH_2 \qquad (IV)$$

in der $R_5$ die oben angegebenen Bedeutungen hat, umsetzt.

Die Kondensation der Verbindungen der Formel VI mit Verbindungen der Formel IV erfolgt in üblicher Weise, vorteilhaft in einem protischen oder aprotischen Lösungsmittel, wie z.B. in einem aliphatischen Halogenkohlenwasserstoff, wie in Dichlormethan, insbesondere Methylenchlorid, oder eines aliphatischen oder cycloaliphatischen Ethers, z.B. Tetrahydrofuran oder auch Dioxan. Ferner können als Lösungsmittel beispielsweise noch Acetonitril, Ethanol oder auch Toluol verwendet werden.

Die Substanzen werden bei einer Temperatur von 25-120°, vorteilhafterweise bei Siedetemperatur des Lösungsmittels in Gegenwart eines basischen Kondensationsmittels wie z.B. Dimethylaminopyridin, Triniederalkylamin, beispielsweise Triethylamin oder auch Chinolin und Pyridin umgesetzt.

Ausgangsverbindungen der Formel VI sind neu und können aus der entsprechenden, vorher erwähnten Hydrazonverbindung der Formel II

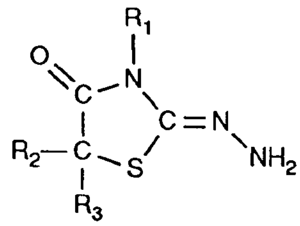

(II)

durch Umsatz mit Schwefelkohlenstoff, $CS_2$ und anschliessender Reaktion mit einem Niederalkyljodid erhalten werden. Der Umsatz einer Verbindung der Formel II mit Schwefelkohlenstoff erfolgt in Gegenwart einer tertiären organischen Base, wie eines Triniederalkylamins, von Hünig'scher Base oder einer organischen Stickstoffbase, wie z.B. Pyridin oder Chinolin. Der nachfolgende Umsatz mit einem Niederalkyljodid erfolgt under Kühlen des erhaltenen Reaktionsgemisches auf eine Temperatur zwischen -10° und +10°C, vorzugsweise auf eine Temperatur 0° bis +5°C.

Nach einem weiteren Verfahren d) lassen sich Verbindungen der Formel I, worin $R_4$ Niederalkyl, Niederalkoxy, Aryl oder Arylniederalkyl bedeutet, herstellen, indem man Verbindungen der Formel VII

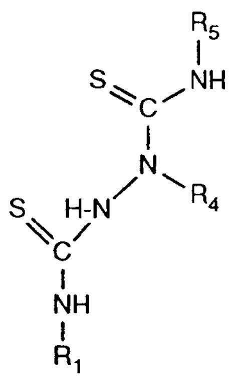

(VII)

worin $R_4$ die oben angegebenen Bedeutungen hat und $R_5$ die unter der Formel I angegebenen Bedeutungen hat, mit einer Halogenessigsäure der Formel VIII

$X\text{-}CH_2COOH$ (VIII)

worin X ein Halogenatom, insbesondere Bromatom bedeutet, zum entsprechenden Thiazolidinon-thiosemicarbazon der Formel I ringschliesst.

Die Umsetzung erfolgt in Gegenwart eines basischen Kondesationsmittels, wie z.B. Natriumacetat bei einer Temperatur zwischen 25-80°C, vorzugsweise bei einer Temperatur zwischen 30-50°C.

Die Kondensation der Verbindungen der Formel VII mit Verbindungen der Formel VIII erfolgt in üblicher Weise in einem protischen oder aprotischen Lösungsmittel, wie z.B. eines aliphatischen oder cycloaliphatischen Ethers, z.B. in Tetrahydrofuran oder auch Dioxan, vorzugsweise jedoch Ethanol und Acetonitril.

Verbindungen der Formel VII lassen sich aus dem entsprechenden Thiosemicarbazid der Formel IX

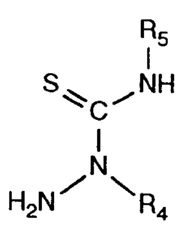

(IX)

durch Umsetzung mit einem Isothiocyanat der Formel X

$R_1\text{-}NCS$ (X)

erhalten.

Die Kondensation der Verbindungen der Formel IX mit Verbindungen der Formel X erfolgt in üblicher Weise in einem protischen oder aprotischen Lösungsmittel, wie z.B. eines aliphatischen Halogenkohlenwasserstoffes, wie in Dichlormethan, insbesondere Methylenchlorid, oder eines aliphatischen oder cycloaliphatischen Ethers, z.B. in Tetrahydrofuran oder auch Dioxan. Ferner kommen als Lösungsmittel beispielsweise noch Acetonitril, Ethanol und Toluol in Frage.

Die Substanzen werden bei einer Temperatur von 25-120°, vorteilhafterweise bei Siede-temperatur des Lösungsmittels in Gegenwart eines basischen Kondensationsmittels wie z.B. Diemthylaminopyridin, Triethylamin oder Pyridin umsetzt.

Verbindungen der Formel IX lassen sich aus den entsprechenden Hydrazinen der Formel XI

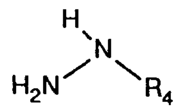

(XI)

durch Umsetzung mit einem Isothiocyanat der Formel III

$R_5$-NCS (III)

erhalten

Die Kondensation der Verbindungen der Formel XI mit Verbindungen der Formel XII erfolgt in üblicher Weise in einem protischen oder aprotischen Lösungsmittel, wie z.B. eines aliphatischen Halogenkohlenwasserstoffes, wie in Dichlormethan, insbesondere Methylenchlorid, oder eines aliphatischen oder cycloaliphatischen Ethers, z.B. in Tetrahydrofuran oder auch Dioxan. Ferner kommen als Lösungsmittel beispielsweise noch Acetonitril, Ethanol und Toluol in Frage.

Die Substanzen werden bei einer Temperatur zwischen -10° bis +30°C, vorzugsweise zwischen 0°C bis +20°C miteinander umgesetzt.

Die nach den Verfahrensvarianten a)-d) erfindungsgemäss erhältlichen als Isomerengemisch vorliegenden Verbindungen der Formel I können gewünschtenfalls in die einzelnen Isomeren aufgetrennt werden und/oder erfindungsgemäss erhältliche freie Verbindungen in ein Salz oder ein erfindungsgemäss erhältliches Salz in eine freie Verbindung oder in ein anderes Salz überführt werden.

Verfahrensgemäss erhältliche Verbindungen können in üblicher Weise in andere Verbindungen der Formel I überführt werden.

Beispielsweise können Verbindungen der Formel I

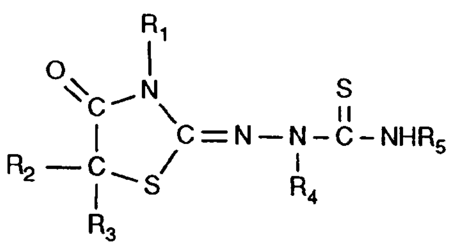

(I)

worin $R_1$, $R_2$, $R_3$ und $R_5$ die oben angegebenen Bedeutungen haben und $R_4$ Wasserstoff bedeutet, mit einem Säurehalogenid der Formel IIIb X-C(=O)-$R_6$ umgesetzt werden, in der X ein Halogenatom bedeutet, um Verbindungen der Formel I zu erhalten, in denen $R_4$ die Gruppe

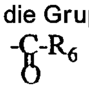

bedeutet.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten

werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-O-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Bei den erfindungsgemassen pharmazeutischen Präparaten, welche die erfindungsgemässe Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Starke-kleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, LacMösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykol, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyethylenglykol oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykol oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Erfindung betrifft ebenfalls die Verwendung der Verbindungen der Formel I, vorzugsweise in Form von pharmazeutischen Präparaten. Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 5 mg bis etwa 1000 mg, insbesondere von etwa 10 mg bis etwa 200 mg zu veranschlagen. Diese kann auf auf einmal gegeben oder auf mehrere, z.B. 2 bis 4 Einzeldosen verteilt werden. Pharmazeutische Präparate in Dosiseinheitsform enthalten somit von etwa 5 mg bis etwa 250 mg, insbesondere von etwa 10 mg bis etwa 50 mg.

Die nachfolgen Beispiele dienen zur Illustration der Erfindung; Temperaturen sind in Celsiusgraden, Drucke in mbar angegeben.

Beispiel 1:

1,0 g 1-(3-Allyl-4-oxo-thiazolidin-2-yliden)-hydrazon und 0,87 g Phenylisothiocyanat werden in 15 ml Ethanol als Lösungsmittel 1 Stunde lang bei Raumtemperatur gerührt. Das gut kristallisierende Produkt wird abfiltriert, mit kaltem Ethanol oder Ether und Petrolether gewaschen und am Hochvakuum getrocknet. Man erhält das kristalline 1-(3-Allyl-4-oxo-thiazolidin-2-yliden)-4-phenyl-thiosemicarbazon mit einem F.P. 156-158°C.

Beispiel 2:

Analog zu Beispiel 1 werden 1 g 1-(3-Allyl-4-oxo-thiazolidin-2-yliden)-hydrazon und 1,38 g p-Bromphenylisothiocyanat in 15 ml Ethanol für 0,5 Stunden auf 50°C erhitzt. Nach Filtration werden analog wie unter Beispiel 1 beschrieben Kristalle des 1-(3-Allyl-4-oxo-thiazolidin-2-yliden)-4-p-bromphenyl-thiosemicarbazones mit einem F.P. 178-180°C erhalten.

Beispiel 3:

Analog zu zu Beispiel 1 werden 0,7 g 1-(3-Allyl-4-oxo-thiazolidin-2-yliden)-hydrazon und 0,7 g p-Fluorphenylisothiocyanat in 15 ml Ethanol für 1 Stunde am Rückfluss gekocht. Nach Filtration werden Kristalle des 1-(3-Allyl-4-oxo-thiazolidin-2-yliden)-4-p-fluorphenyl-thiosemicarbazons mit einem F.P. 152-154°C erhalten.

Beispiel 4:

0,65 g 1-(3-Allyl-4-oxo-thiazolidin-2-yliden)-hydrazono-1-dithioameisensäuremethylesteramid und 0,27 g 4-Aminophenol werden mit einer katalytischen Menge Dimethylaminopyridin in 20 ml Ethanol 5 Stunden lang unter Rückfluss bis zur Vervollständigung der Reaktion erwärmt. Nach dem Abkühlen wird das erhaltene kristallisierende Produkt 1-(3-Allyl-4-oxo-thiazolidin-2-yliden)-4-p-hydroxyphenyl-thiosemicarbazon abfiltriert, F.P. 212-214°C.

Beispiel 5:

Analog zu Beispiel 1 werden 0,7 g 1-(3-Allyl-4-oxo-thiazolidin-2-yliden)-hydrazon und 0,6 ml Pentafluorphenylisothiocyanat in 15 ml Ethanol für 1 Stunde am Rückfluss gekocht. Nach dem Abkühlen werden Kristalle des erhaltenen 1-(3-Allyl-4-oxo-thiazolidin-2-yliden)-4-pentafluorophenylthiosemicarbazons abfiltriert, F.P. 176-178°C.

Beispiel 6:

Analog zu Beispiel 1 werden 1 g 1-(3-Allyl-4-oxo-thiazolidin-2-yliden)-hydrazon und 1 g Tolylisothiocyanat in 20 ml Ethanol für 1 Stunde am Rückfluss gekocht. Nach Filtration werden Kristalle des 1-(3-Allyl-4-oxo-thiazolidin-2-yliden)-4-tolyl-thiosemicarbazons erhalten, F.P. 173-175°C.

Beispiel 7:

Analog zu Beispiel 4 werden 0,75 g 1-(3-Allyl-4-oxo-thiazolidin-2-yliden)-hydrazono-1-dithioameisensäuremethylesteramid und 0,4 g 4-Aminobenzonitril mit einer katalytischen Menge Dimethylaminopyridin in 20 ml Ethanol für 5 Stunden am Rückfluss cyanophenyl-thiosemicarbazons erhalten, F.P. 192-193°C.

Beispiel 8:

Analog zu Beispiel 1 werden 2 g 1-(3-Allyl-4-oxo-thiazolidin-2-yliden)-hydrazon und 1,75 g Benzylisothiocyanat in 30 ml Ethanol 2 Stunden am Rückfluss gekocht. Nach Filtration werden Kristalle des 1-(3-Allyl-4-oxo-thiazolidin-2-yliden)-4-benzylthiosemicarbazons erhalten, F.P. 120°C.

Beispiel 9:

Analog zu Beispiel 1 werden 1 g 1-(3-Allyl-4-oxo-thiazolidin-2-yliden)-hydrazon und 1,35 g Thiophen-2-methylisothiocyanat in 20 ml Ethanol 0,5 Stunden am Rückfluss gekocht. Nach Filtration werden Kristalle des 1-(3-Allyl-4-oxo-thiazolidin-2-yliden)4-(thiophen-2-methyl)-thiosemicarbazons erhalten, F.P. 114-115°C.

Beispiel 10:

Analog zu Beispiel 1 werden 0,5 g 1-(3-Allyl-4-oxo-thiazolidin-2-yliden)-hydrazon und 1,0 g Furan-2-methylisothiocyanat in 20 ml Ethanol 0,5 Stunden am Rückfluss gekocht. Nach Filtration werden Kristalle des 1-(3-Allyl-4-oxo-thiazolidin-2-yliden)-4-(furan-2-methyl)-thiosemicarbazons erhalten, F.P. 120-122°C .

Beispiel 11:

Analog Beispiel 1 werden 1,6 g 1-(3-Allyl-4-oxo-thiazolidin-2-yliden)-hydrazon und 1,4 g Tetrahydrofuran-2-methylisothiocyanat in 20 ml Ethanol 1 Stunde am Rückfluss gekocht. Nach Filtration und zweimaliger Umkristallisation aus Ethanol/Methylenchlorid werden Kristalle des 1-(3-Allyl-4-oxo-thiazolidin-2-yliden)-4-(tetrahydrofuran-2-methyl)-thiosemicarbazons erhalten, F.P. 120-122°C.

Beispiel 12:

Analog zu Beispiel 4 werden 0,7 g 2-(3-Allyl-4-oxo-thiazolidin-2-yliden)-hydrazono-1-dithioameisensäuremethylesteramid und 0,32 g 2-Picolylamin in 20 ml Ethanol mit einer katalytischen Menge Dimethylaminopyridin 3 Stunden am Rückfluss gekocht. Nach Filtration werden Kristalle des 1-(3-Allyl-4-oxo-thiazolidin-2-yliden)-4-(2-picolyl)-thiosemicarbazons erhalten, F.P. 177-178°C.

Beispiel 13:

Analog zu Beispiel 1 werden 1 g 1-(3-Allyl-4-oxo-thiazolidin-2-yliden)-hydrazon und 1,1 g Phenylethylisothiocyanat in 20 ml Ethanol 1 Stunde auf 50°C erhitzt. Nach Abkühlen und Filtration werden Kristalle des 1-(3-Allyl-4-oxo-thiazolidin-2-yliden)-4-phenylethyl-thiosemicarbazons erhalten, F.P. 140-141°C.

Beispiel 14:

Analog zu Beispiel 1 werden 1,7 g 1-(3-Allyl-4-oxo-thiazolidin-2-yliden)-hydrazon und 1,4 g Glycinethylester-isocyanat in 25 ml Ethanol 1,5 Stunden am Rückfluss gekocht. Nach dem Abkühlen wird das Gemisch eingeengt. Das Rohprodukt wird aus Methylenchlorid/Ether/Petroleter umkristallisiert. Man erhält das 1-(3-Allyl-4-oxo-thiazolidin-2-yliden)-4-glycinylethylester-thiosemicarbazon, F.P. 131-132°C.

Beispiel 15:

Analog zu Beispiel 1 werden 5 g 1-(5,5-Dimethyl-3-methallyl-4-oxo-thiazolidin-2-yliden)-hydrazon und 3,2 g Glycinethylester-isothiocyanat in 80 ml Ethanol 3 Stunden am Rückfluss gekocht. Nach Abkühlen und Filtration wird das erhaltene Roh-produkt mit Methylenchlorid chromatographiert an Kieselgel und aus Ether/Petrolether umkristallisiert. Man erhält das 1-(5,5-Dimethyl-3-methallyl-4-oxo-thiazolidin-2-yliden)-4-glycinylethylester-thiosemicarbazon, F.P. 112-113°C.

Beispiel 16:

Analog zu Beispiel 4 werden 0,65 g 2-(3-Allyl-4-oxo-thiazolidin-2-yliden)-hydrazono-1-dithioameisensäuremethylesteramid und 0,3 ml Aminoacetaldehyddimethyl-acetal in 20 ml Ethanol mit einer katalytischen Menge Dimethylaminopyridin 12 Stunden am Rückfluss gekocht. Nach dem Abziehen des Lösungsmittels werden aus Methylenchlorid/Ether Kristalle des 1-(3-Allyl-4-oxo-thiazolidin-2-yliden)-4-(2',2'-dimethoxyethyl)-thiosemicarbazons erhalten, F.P. 113-114°C.

Beispiel 17:

1,9 g 4-Allyl-1-methyl-thiosemicarbazon-1-methyl-amino-thioamid werden mit 1,5 g Bromessigsäure und 1,75 g wasserfreiem Natriumacetat in 20 ml Ethanol als Lösungsmittel vemischt und 6 Stunden bei Raumtemperatur und anschliessend weitere 14 Stunden bei 50°C gerührt. Nach dem Abkühlen wird das Lösungsmittel am Rotations-verdampfer abgezogen und das erhaltene Rohprodukt an Kieselgel mit Methylenchlorid chromatographiert und anschliessend aus Methylenchlorid/Ether umkristallisiert. Man erhält kristallines 1-(3-Allyl-4-oxo-thiazolidin-2-yliden)-2,4-dimethylthiosemicarbazon mit einem F.P. 142-143°C.

Beispiel 18:

Analog zu Beispiel 4 werden 1.3 g 2-(3-Allyl-4-oxo-thiazolidin-2-yliden)-hydrazono-1-dithioameisensäremethylesteramid und 0.7 g 2-Aminobenzylalkohol in 20 ml Ethanol mit einer katalytischen Menge Dimethyaminopyridin 8 Stunden am Rückfluss gekocht. Nach dem Abziehen des Lösungsmittels werden aus Methylenchlorid/Ether Kristalle des 1-(3-Allyl-4-oxothiazolidin-2-yliden)-4-hydroxymethylphenyl-thiosemicarbazons erhalten, F.P. 146-147°C.

Beispiel 19:

Analog zu Beispiel 4 werden 1,0 g 2-(3-Allyl-4-oxo-thiazolidin-2-yliden)-hydrazono-1-dithioameisensäuremethylesteramid und 0.58 g 3,4-Methylendioxyanilin in hydrazono-1-dithioameisensäuremethylesteramid und 0.58 g 3,4-Methylendioxyanilin in 20 ml Ethanol mit einer katalytischen Menge Dimethylaminopyridin 7 Stunden am Rückfluss gekocht. Nach dem Abziehen des Lösungsmittels werden aus Methylenchlorid/Ether Kristalle des 1-(3-Allyl-4-oxo-thiazolidin-2-yliden)-4-(3,4-methylendioxyphen-1-yl-thiosemicarbazons erhalten, F.P. 165-167°C.

Beispiel 20:

Analog zu Beispiel 4 werden 3.5 g 2-(3-Allyl-4-oxo-thiazolidin-2-yliden)-hydrazono-1-dithioameisensäuremethylesteramid und 2.1 g D,L-Alaninmethylesterhydrochlorid in 20 ml Ethanol und 2.1 ml Triethylamin mit einer katalytischen Menge Dimethylaminopyridin 8 Stunden am Rückfluss gekocht. Nach dem Abkühlen wird das Lösungsmittel am Rotationsverdampfer abgezogen und das erhaltene Rohprodukt an Kieselgel mit Methylenchlorid/Aceton chromatographiert und anschliessend aus Methylenchlorid/Ether umkristallisiert. Man erhält kristallines 1-(3-Allyl-4-oxo-thiazolidin-2-yliden)-4-(propionäureethylester-2-yl)-thiosemicarbazon, F.P. 110-111°C.

Beispiel 21:

Bei 0°C werden zu einer Suspension von 1,35 g Kaliumrhodanid in 30 ml Acetonitril unter Rühren 1,4 ml Isobuttersäurechlorid zugetropft und auf Raumtemperatur aufwärmen gelassen. Nach 30 Minuten werden 2 g 1-(3-Allyl-4-oxo-thiazolidin-2-yliden)-hydrazon hinzugetropft und die erhaltene Mischung 3 Stunden bis zur Vervollständigung der Reaktion bei Raumtemperatur gerührt. Das Lösungsmittel wird am Rotationsverdampfer

abgezogen und der erhaltene Rückstand in Methylenchlorid aufgenommen, mit Wasser gewaschen, über Magnesiumsulfat getrocknet und auf 2/3 seines Volumens eingeengt. Nach Zugabe von Ether kristallisiert das erhaltene Produkt aus und wird nach dem Abfiltrieren aus Methylenchlorid/Ether umkristallisiert. Man erhält reine Kristalle des 1-(3-Allyl-4-oxo-thiazolidin-2-yliden)-4-(2-methyl-propionyl)-thiosemicarbazons mit F.P. 165-166°C.

Beispiel 22:

1,2 g Benzoylisothiocyanat werden in 30 ml Acetonitril vorgelegt und 2 g 1-(5,5-Dimethyl-3-methallyl-4-oxo-thiazolidin-2-yliden)-hydrazon in wenig Acetonitril zugetropft. Das Gemisch wird unter Rühren 1,5 Stunden under Rückfluss erwärmt. Nach dem Abkühlen auf Raumtemperatur wird der Festkpörper abfiltriert und mit Ether/Petrolether gewaschen und das erhaltene Rohprodukt an Kieselgel mit Methylenchlorid chromatographiert. Man erhält das 1-(5,5-Dimethyl-3-methallyl-4-oxo-thiazolidin-2-yliden)-4-benzoyl-thiosemicarbazon mit einem F.P. 152-155°C. bei 0°C mit 1,4 ml Thiophen-2-carbonsäurechlorid versetzt und anschliessend 2 g 1-(3-Allyl-4-oxo-thiazolidin-2-yliden)-hydrazon zugegeben. Das Gemisch wird anschliessend 3,5 Stunden bei Raumtemperatur gerührt. Nach Aufarbeitung und anschliessender Umkristallisation aus Methylenchlorid/Ether werden Kristalle des 1-(3-Allyl-4-oxo-thiazolidin-2-yliden)-4-(thiophen-2-carbonyl)-thiosemicarbazons mit einem F.P. 185°C erhalten.

Beispiel 24:

Analog zu Beispiel 21 werden 1,35 g Kaliumrhodanid in 30 ml Acetonitril bei 0°C mit 1,73 ml Phenylacetylchlorid versetzt und anschliessend 2,0 g 1-(3-Allyl-4-oxo-thiazolidin-2-yliden)-hydrazon zugegeben. Das Gemisch wird anschliessend 3 Stunden bei Raumtemperatur gerührt. Nach Aufarbeitung und anschliessender Umkristallisation aus Methylenchlorid/Ether werden Kristalle des 1-(3-Allyl-4-oxo-thiazolidin-2-yliden)-4-phenylacetyl-thiosemicarbazons F.P. 190-191°C erhalten.

Beispiel 25:

Analog zu Beispiel 21 werden 0,85 g Ammoniumrhodanid in 30 ml Acetonitril bei 0°C mit 1,4 ml Chlorameisensäurephenylester versetzt und anschliessend 1,7 g 1-(3-Allyl-4-oxo-thiazolidin-2-yliden)-hydrazon zugegeben. Das Gemisch wird anschliessend 15 Minuten bei Raumtemperatur gerührt. Nach Aufarbeitung und anschliessender Umkristallisation aus Ethanol/Methylenchlorid werden Kristalle des 1-(3-Allyl-4-oxo-thiazolidin-2-yliden)-4-phenoxycarbonyl-thiosemicarbazons mit einem F.P. 173°C (Zersetzung) erhalten.

Beispiel 26:

Analog zu Beispiel 21 werden 0,7 g Kaliumrhodanid in 30 ml Acetonitril bei 0°C mit 0,9 ml Chlorameisensäurebenzylester versetzt und anschliessend 1 g 1-(3- Allyl-4-oxo-thiazolidin-2-yliden)-hydrazon zugegeben. Das Gemisch wird anschliessend 3 Stunden bei Raumtemperatur gerührt. Nach Aufarbeitung and anschliessender Umkristallisation aus Ethanol/Methylenchlorid werden Kristalle des 1-(3-Allyl-4-oxo-thiazolidin-2-yliden)-4-benzyloxycarbonyl-thiosemicarbazons mit einem F.P. 165°C erhalten.

Beispiel 27:

Zu einer Mischung von 2,4 g 1-(3-Allyl-4-oxo-thiazolidin-2-yliden)-4-methyl-thiosemicarbazon und 1,5 ml Triethylamin in 20 ml Methylenchlorid werden unter Rühren bei 0°C mit 0.8 ml Acetylchlorid versetzt und anschliessend auf Raumtemperatur aufwärmen gelassen. Nach 1 stündigem Rühren bei Raumtemperatur wird das Gemisch mit Wasser extrahiert und die organische Phase über Magnesiumsulfat getrocknet eingeengt. Das Rohprodukt wird aus Ether/Methylenchlorid umkristallisiert und das net eingeengt. Das Rohprodukt wird aus Ether/Methylenchlorid umkristallisiert und das abfiltrierte Produkt am Hochvakuum getrocknet. Man erhält Kristalle des 1-(3-Allyl-4-oxo-thiazolidin-2-yliden)-2-acetyl-4-methyl-thiosemicarbazons mit einem F.P. 125°C.

Beispiel 28:

Analog zu Beispiel 27 werden 1,2 g 1-(3-Allyl-4-oxo-thiazolidin-2-yliden)-4-thiosemicarbazon und 1,5 ml Triethylamin in 20 ml Methylenchlorid unter Rühren bei 0°C mit 1,3 ml Benzoylchlorid versetzt. Nach Aufarbeitung und anschliessender Kristallisation aus Ether werden Kristalle des 1-(3-Allyl-4-oxo-thiazolidin-2-yliden)-2-benzoyl-4-methyl-thiosemicarbazon mit einem F.P. 113°C erhalten.

Beispiel 29:

Analog zu Beispiel 21 werden 1,0 g Kaliumrhodanid in 30 ml Acetonitril bei 0°C mit 1,2 ml Chlorameisensäureallylester versetzt und anschliessend 1,2 g 1-(3-Allyl-4-oxo-thiazolidin-2-yliden)-hydrazon zugegeben. Das Gemisch wird anschliessend 3 Stunden bei Raumtemperatur gerührt. Nach Aufarbeitung und anschliessender Umkristallisation aus Ethanol/Methylenchlorid werden Kristalle des 1-(3-Allyl-4-oxo-thiazolidin-2-yliden)-4-allyloxycarbonyl-thiosemicarbazons erhalten.

Beispiel 30:

Analog zu Beispiel 21 werden 0.85 g Ammoniumrhodanid in 30 ml Acetonitril bei O°C mit 1.82 g Zimtsäurechlorid versetzt und anschliessend 1.7 g 1-(3-Allyl-4-oxo-thiazolidin-2-yliden)-hydrazon dazu gegeben. Das erhaltene Gemisch wird anschliessend 3 Stunden bei Raumtemperatur gerührt. Nach Aufarbeitung und anschliessender Umkristallisation aus Methylenchlorid/Ether wird zuerst acyliertes Hydrazon als Nebenprodukt abgetrennt und aus der Mutterlauge werden nach Zugabe einer weiteren Portion Ether Kristalle des 1-(3-Allyl-4-oxo-thiazolidin-2-yliden)-4-cinnamoyl-thiosemicarbazons erhalten, F.P. 142-144°C.

Beispiel 31:

Tabletten, enthaltend je 10 mg Wirkstoff, können wie folgt hergestellt werden:

Zusammensetzung (10000 Tabletten)

Zusammensetzung (10000 Tabletten)

| | |
|---|---|
| Wirkstoff | 100,0 g |
| Lactose | 450,0 g |
| Kartoffelstärke | 350,0 g |
| Gelatine | 10,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 100,0 mg Gewicht und 10,0 mg Wirkstoffgehalt, die gewünschtenfalls mit Teilkerben zur feineren Anpassung der Dosierung versehen sein können.

Beispiel 32:

Gelatinesteckkapseln, enthaltend 20 mg Wirkstoff, können z.B. folgendermassen hergestellt werden:

Zusammensetzung (für 1000 Kapseln)

| | |
|---|---|
| Wirkstoff | 20,0 g |
| Lactose | 240,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 300 mg der erhaltenen Formulierung in Gelatinesteckkapseln der Grösse 0 abgefüllt.

Beispiel 33:

Gelatinesteckkapseln, enthaltend 100 mg Wirkstoff, können z.B. folgendermassen hergestellt werden:

Zusammensetzung (für 1000 Kapseln)

| | |
|---|---|
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteck-kapseln der Grösse 0 abgefüllt.

Beispiel 34:

Lacktabletten, enthaltend je 50 mg Wirkstoff, können wie folgt hergestellt werden:

Zusammensetzung (für 1000 Lacktabletten)

| | |
|---|---|
| Wirkstoff | 50,0 g |
| Lactose | 100,0 g |
| Maisstärke | 70,0 g |
| Talk | 10,0 g |
| Calciumstearat | 2,0 g |
| Hydroxypropylmethylcellulose | 2,36 g |
| Schellack | 0,64 g |
| Wasser | q.s. |
| Methylenchlorid | q.s |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 240 mg) verpresst und diese mit einer Lösung der Hydroxypropylmethylcellulose und des Schellacks in Methylenchlorid lackiert; Endgewicht der Lack-tablette: 283 mg.

Beispiel 35:

Eine 0,2%-ige Injektions- oder Infusionslösung eines Wirkstoffes kann beispielsweise folgendermassen hergestellt werden:

Zusammensetzung (für 1000 Ampullen)

| | |
|---|---|
| Wirkstoff | 5,0 g |
| Natriumchlorid | 22,5 g |
| Phosphatpuffer pH=7,4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

Beispiel 36:

1 %-ige Salbe (O/W-Emulsion), einen Wirkstoff enthaltend, mit folgender Zusammensetzung:

| | |
|---|---|
| Wirskstoff | 1,0 g |
| Cetylalkohol | 3,0 g |
| Glycerin | 6,0 g |
| Methylparaben | 0,18 g |
| Propylparaben | 0,05 g |
| Arlacel 60 | 0,6 g |
| Tween 60 | 4,4 g |
| Stearinsäure | 9,0 g |
| Isopropylpalmitat | 2,0 g |
| Paraffinöl dickflüssig | 10,0 g |
| Wasser entmin. q.s. ad | 100,0 g |

Beispiel 37:

1%-iges Gel, einen Wirkstoff enthaltend, mit folgender Zusammensetzung:

| | |
|---|---|
| Wirkstoff | 1,0 g |
| Carbopol 934 P | 1,0 g |
| Glycerin | 3,0 g |

| | |
|---|---|
| Isopropanol | 25,0 g |
| Softigen® 767 | 0,2 g |
| Wasser entmin. q.s. ad | 100,0 g |

**Patentansprüche**

1. Verbindungen der Formel I

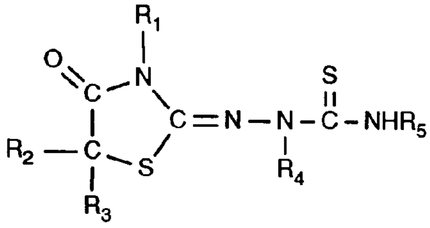

(I)

worin $R_1$ Niederalkyl, Niederalk-2-en-1-yl oder auch Niederalk-2-in-1-yl bedeutet, $R_2$ und $R_3$ unabhängig voneinander Wasserstoff oder Niederalkyl bedeuten oder gemeinsam für Niederalkyliden stehen, $R_4$ Wasserstoff, Niederalkyl, Niederalkoxy, Aryl oder Arylniederalkyl oder die Gruppe -C(=O)-$R_6$ bedeutet,

in der $R_6$ für Niederalkyl, Aryl, Heteroaryl, Aryloxy, Arylniederalkoxy oder Niederalk-2-en-lyloxy steht, $R_5$ Aryl, Arylniederalkyl, ungesättigtes oder gesättigtes Heterocyclylniederalkyl, Niederalkoxycarbonylniederalkyl oder die Gruppe -C(=O)-$R_7$ bedeutet, in der $R_7$ für Niederalkyl, Aryl, Arylniederalkyl, Arylniederalkenyl, Heteroaryl, Aryloxy, Arylniederalkoxy oder Niederalk-2-en-1-yloxy steht oder $R_5$ auch Niederalkyl, Niederalk-2-en-1-yl oder Niederalk-2-in-1-yl bedeutet, wenn $R_4$ verschieden von Wasserstoff ist, und ihre Salze.

**2.** Verbindungen gemäss Anspruch 1 der Formel I, worin $R_1$ $C_1$-$C_4$-Alkyl, $C_3$-$C_5$-Alk-2-en-1-yl oder $C_3$-$C_5$-Alk-2-in-1-yl bedeutet, $R_2$ und $R_3$ unabhängig voneinander Wasserstoff, gleiche $C_1$-$C_4$-Alkyl oder Phenylniederalkyl oder die Gruppe -C(=O)-$R_6$ bedeutet in der $R_6$ für $C_1$-$C_4$-Akyl, Phenyl, Phenylniederalkyl Naphthyl, Pyridyl, Thienyl, Pyrryl, Furyl, Phenyloxy, Phenyl-$C_1$-$C_4$-alkoxy oder $C_3$-$C_5$-Alk-2-en-1-yloxy steht, $R_5$ Phenyl, Naphthyl, Phenylniederalkyl, Pyridyl-, Thienyl-, Pyrryl- oder Furyl-, Pyrrolidinyl-, Tetrahydrofuranyl- oder Tetrahydropyranylniederalkyl oder $C_1$-$C_4$-Alkoxy-carbonyl-$C_1$-$C_4$-alkyl oder die Gruppe -C(=O)-$R_7$ bedeutet, in der $R_7$ die für $R_4$ angegebenen Bedeutungen von $R_5$ hat oder auch Phenylniederalkyl oder Phenylniederalkenyl bedeutet oder $R_5$ auch $C_1$-$C_4$-Alkyl, $C_3$-$C_5$-Alk-2-en-1yl oder $C_3$-$C_5$-Alk-2-in-1yl bedeutet, wenn $R_4$ verschieden von Wasserstoff ist, und ihre pharmazeutisch verwendbaren Salze.

**3.** Verbindungen gemäss Anspruch 1 der Formel I, worin $R_1$ $C_3$-$C_5$-Alk-2-en-1-yl, wie Allyl oder Methallyl oder $C_3$-$C_5$-Alk-2-in-1-yl, wie Prop-2-in-1-yl bedeutet. $R_2$ und $R_3$ beide Wasserstoff oder gleiche $C_1$-$C_4$-Alkyl, wie Methylgruppen bedeuten, $R_4$ Wasserstoff, $C_1$-$C_4$-Alkyl, wie Methyl oder Ethyl, $C_1$-$C_2$-Alkoxy, wie Methoxy oder Ethoxy, Phenyl, Phenylniederalkyl, wie z.B. Benzyl oder Phenylethyl oder die Gruppe -C(=O)-$R_5$ bedeutet, in der $R_6$ für $C_1$-$C_4$-Alkylgruppen, wie z.B. die Methylgruppe, Phenyl, Pyridyl, Thienyl, Phenyloxy, Benzyloxy $C_3$-$C_5$-Alk-2-en-1-yloxy, wie Allyl- oder Methallyloxy steht und $R_5$ Phenyl, Phenylniederalkyl, wie z.B. Benzyl oder Phenylethyl, Pyridyl-, Thienyl-, Pyrryl-, Furyl- Pyrrolidinyl-, Tetrahydrofuranyl- oder Tetrahydropyranylniederalkyl, wie z.B. Pyridyl-, Thienyl-, Pyrryl- oder Furyl-, Pyrrolidinyl-, Tetrahydrofuranyl- oder Tetrahydropyranylmethyl, $C_1$-$C_4$-Alkoxycarbonyl-$C_1$-$C_4$-alkyl, wie z.B. Methoxy- oder ethoxycarbonylmethyl- oder -ethyl oder die Gruppe -C(=O)-$R_7$ bedeutet, in der $R_7$ die für $R_4$ angegebenen Bedeutungen von $R_6$ hat oder auch Benzyl oder Phenylallyl bedeutet oder $R_5$ auch $C_1$-$C_4$-Alkyl, wie z.B. Methyl, $C_3$-$C_5$-Alkyl-2-en-1-yl, wie z,B. Allyl oder Methallyl, $C_3$-$C_5$-Alk-2-in-1-yl, wie z.B. Pro-2-in-lyl bedeutet, wenn $R_4$ verschieden von Wasserstoff ist und ihre pharmazeutisch verwendbare Salze.

**4.** Verbindungen gemäss Anspruch 1 der Formel I, worin $R_1$ Allyl oder Methallyl, $R_2$ und $R_3$ beide Wasserstoff und $R_4$ Wasserstoff, Methyl oder Methoxy oder die Gruppe -C(=O)-$R_5$ bedeuten, in der $R_5$ für Methyl, Phenyl, Phenyloxy, Benzyloxy oder Allyloxy steht und $R_5$ Phenyl, Benzyl, Pyridyl-, Furyl- oder Tetrahydropyranylmethyl oder Methoxycarbonylmethyl oder die Gruppe -C(=O)-$R_7$ bedeutet, in der $R_7$ die für $R_4$ angegebenen Bedeutungen von $R_6$ hat oder auch Benzyl oder Phenylallyl bedeutet, oder $R_5$ auch Methyl, Allyl, Methallyl, Prop-2-in-1yl bedeutet, wenn $R_4$ verschieden von Wasserstoff ist, und ihre pharmazeutisch verwendbare Salze.

**5.** 1-(3-Allyl-4-oxo-thiazolidin-2-yliden)-4-phenyl-thiosemicarbazon oder ein pharmazeutisch verwendbares Salz davon.

**6.** 1-(3-Allyl-4-oxo-thiazolidin-2-yliden)-4-p-bromphenyl-thiosemicarbazon oder ein pharmazeutisch verwendbares Salz davon.

**7.** 1-(3-Allyl-4-oxo-thiazolidin-2-yliden)-4-p-fluorphenyl-thiosemicarbazon oder ein pharmazeutisch verwendbares Salz davon.

**8.** 1-(3-Allyl-4-oxo-thiazolidin-2-yliden)-4-pentafluorophenyl-thiosemicarbazon oder ein pharmazeutisch verwendbares Salz davon.

**9.** 1-(3-Allyl-4-oxo-thiazolidin-2-yliden)-4-tolyl-thiosemicarbazon oder ein pharmazeutisch verwendbares Salz davon.

**10.** 1-(3-Allyl-4-oxo-thiazolidin-2-yliden)-4-p-cyanophenyl-thiosemicarbazon oder ein pharmazeutisch verwendbares Salz davon.

**11.** 1-(3-Allyl-4-oxo-thiazolidin-2-yliden)-4-benzyl-thiosemicarbazon oder ein pharmazeutisch verwendbares Salz davon.

**12.** 1-(3-Allyl-4-oxo-thiazolidin-2-yliden)-4-(thiophen-2-methyl)-thiosemicarbazon oder ein pharmazeutisch verwendbares Salz davon.

**13.** 1-(3-Allyl-4-oxo-thiazolidin-2-yliden)-4-(furan-2-methyl)-thiosemicarbazon oder ein pharmazeutisch verwendbares Salz davon.

**14.** 1-(3-Allyl-4-oxo-thiazolidin-2-yliden)-4-(tetrahydrofuran-2-methyl)-thiosemicarbazon oder ein pharmazeutisch verwendbares Salz davon.

**15.** 1-(3-Allyl-4-oxo-thiazolidin-2-yliden)-4-(2-picolyl)-thiosemicarbazon oder ein pharmazeutisch verwendbares Salz davon.

**16.** 1-(3-Allyl-4-oxo-thiazolidin-2-yliden)-4-phenylethyl-thiosemicarbazon oder ein pharmazeutisch verwendbares Salz davon.

**17.** 1-(3-Allyl-4-oxo-thiazolidin-2-yliden)-4-glycinylethylester-thiosemicarbazon oder ein pharmazeutisch verwendbares Salz davon.

**18.** 1-(5,5-Dimethyl-3-methallyl-4-oxo-thiazolidin-2-yliden)-4-glycinylethylester-thiosemicarbazon oder ein pharmazeutisch verwendbares Salz davon.

**19.** 1-(3-Allyl-4-oxo-thiazolidin-2-yliden)-4-(2,2-dimethoxyethyl)-thiosemicarbazon oder ein pharmazeutisch verwendbares Salz davon.

**20.** 1-(3-Allyl-4-oxo-thiazolidin-2-yliden)-2,4-dimethyl-thiosemicarbazon oder ein pharmazeutisch verwendbares Salz davon.

**21.** 1-(3-Ally-4-oxo-thiazolidin-2-yliden)-4-hydroxymethyphenyl-thiosemicarbazon oder ein pharmazeutisch verwendbares Salz davon.

**22.** 1-(3-Allyl-4-oxothiazolidin-2-yliden)-4-(3,4-methylendioxyphen-1-yl-thiosemicarbazon oder ein pharmazeutisch verwendbares Salz davon.

**23.** 1-(3-Allyl-4-oxo-thiazolidin-2-yliden)-4-(propionsäureethylester-2-yl)-thiosemicarbazon oder ein pharmazeutisch verwendbares Salz davon.

**24.** 1-(3-Allyl-4-oxo-thiazolidin-2-yliden)-4-(2-methyl-propionyl)-thiosemicarbazon oder ein pharmazeutisch verwendbares Salz davon.

**25.** 1-(5,5-Dimethyl-3-methallyl-4-oxo-thiazolidin-2-yliden)-4-benzoyl-thiosemicarbazon oder ein pharmazeutisch verwendbares Salz davon.

**26.** 1-(3-Allyl-4-oxo-thiazolidin-2-yliden)-4-(thiophen-2-carbonyl)-thiosemicarbazon oder ein pharmazeutisch verwendbares Salz davon.

**27.** 1-(3-Allyl-4-oxo-thiazolidin-2-yliden)-4-phenylacetyl-thiosemicarbazon oder ein pharmazeutisch verwendbares Salz davon.

**28.** 1-(3-Allyl-4-oxo-thiazolidin-3-yliden)-4-phenoxycarbonyl-thiosemicarbazon oder ein pharmazeutisch verwendbares Salz davon.

**29.** 1-(3-Allyl-4-oxo-thiazolidin-2-yliden)-4-benzyloxycarbonyl-thiosemicarbazon oder ein pharmazeutisch verwendbares Salz davon.

**30.** 1-(3-Allyl-4-oxo-thiazolidin-2-yliden)-2-acetyl-4-methyl-thiosemicarbazon oder ein pharmazeutisch verwendbares Salz davon.

**31.** 1-(3-Allyl-4-oxo-thiazolidin-2-yliden)-4-p-hydroxyphenyl-thiosemicarbazon oder ein pharmazeutisch verwendbares Salz davon.

**32.** 1-(3-Allyl-4-oxo-thiazolidin-2-yliden)-2-benzoyl-4-methyl-thiosemicarbazon oder ein pharmazeutisch verwendbares Salz davon.

**33.** 1-(3-Allyl-4-oxo-thiazolidin-2-yliden)-4-allyloxycarbonyl-thiosemicarbazon oder ein pharmazeutisch verwendbares Salz davon.

**34.** 1-(3-Allyl-4-oxo-thiazolidin-2-yliden)-4-cinnamoyl-thiosemicarbazon oder ein pharmazeutisch verwendbares Salz davon.

**35.** Verbindungen gemäss einem der Ansprüche 1 bis 34 zur Anwendung in einem Verfahren zur therapeutischen Behandlung des menschlichen oder tierischen Körpers.

**36.** Pharmazeutische Präparate, neben üblichen pharmazeutischen Hilfsstoffen als pharmazeutischen Wirkstoff enthaltend eine Verbindung gemäss einem der Ansprüche 1 bis 34 in freier Form oder in pharmazeutisch verwendbarer Salzform.

**37.** Verfahren zur Herstellung neuer Verbindungen der Formel I gemäss Anspruch 1, dadurch gekennzeichnet, dass man

a) eine Verbindung der Formel II

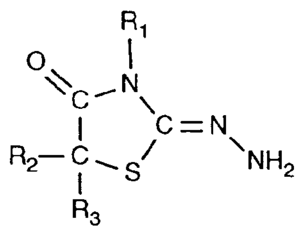

(II)

in der $R_1$, $R_2$ und $R_3$ die oben angegebenen Bedeutungen haben, mit einer Isothiocyanatverbindung der Formel III

$R_5$-NCS (III)

umsetzt, oder

b) dass man eine Verbindung der Formel II mit einer Verbindung der Formel V

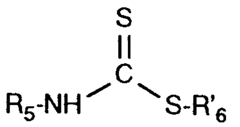

(V)

in der $R_5$ die oben angegebene Bedeutung hat und $R'_6$ Niederalkyl bedeutet, umsetzt, oder

c) dass man eine Verbindung der Formel VI

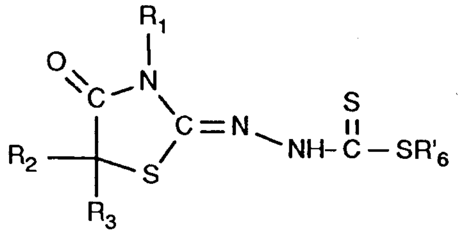

(VI)

in der $R_1$, $R_2$ und $R_3$ die oben angegebenen Bedeutungen haben und $R'_6$ einen Niederalkylrest bedeutet, mit entsprechenden Aminen der oben angegebenen Formel IV

$R_5$-$NH_2$ (IV)

in der $R_5$ die oben angegebenen Bedeutungen hat, umsetzt, oder
d) dass man eine Verbindung der Formel

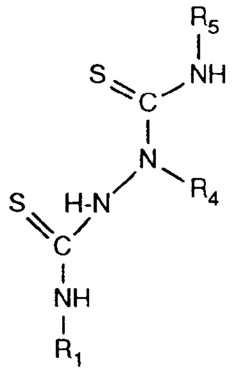

(VII)

worin $R_4$ Niederalkyl, Niederalkoxy, Aryl oder Arylniederalkyl bedeutet und $R_5$ die unter der Formel I angegebenen Bedeutungen hat, mit einer Halogenessigsäure der Formel VIII

$X\text{-}CH_2COOH$ (VIII)

umsetzt, und gewünschtenfalls, wenn man Verbindungen der Formel I, in der $R_4$ die Gruppe

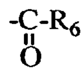

bedeutet, erhalten will, Verbindungen der Formel I,

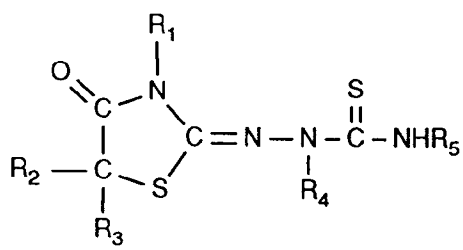

(I)

worin $R_1$, $R_2$, $R_3$ und $R_5$ die oben angegebenen Bedeutungen haben und $R_4$ Wasserstoff bedeutet mit einem Säurehalogenid der Formel IIIb

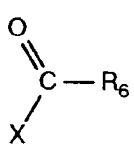

(IIIb)

worin $R_6$ die oben angegebenen Bedeutungen hat und X ein Halogenatom bedeutet, umsetzt und gewünschtenfalls ein erfindungsgemäss erhältliches Isomerengemisch in die individuellen Isomeren auftrennt und das gewünschte Isomere isoliert und/oder eine erfindungsgemäss erhältliche freie Verbindung in ein Salz oder ein erfindungsgemäss erhältliches Salz in die freie Verbindung oder in ein anderes Salz überführt.

**38.** Das Verfahren der Beispiele, verfahrensgemäss verwendete neue Ausgangsstoffe, verfahrensgemäss gebildete neue Zwischenprodukte und verfahrensgemäss erhältliche neue Endstoffe.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung: EP 93 81 0382

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| X | CHEMICAL ABSTRACTS, vol. 61, no. 10, 9. November 1964, Columbus, Ohio, US; abstract no. 12008a, Seite 12007 ; * Zusammenfassung * & JP-B-64 010 345 (TAKEDA) 11. Juni 1964 | 1-4,35, 36 | C07D277/54<br>A61K31/425<br>C07D417/12 |
| A | DE-A-2 035 419 (J.R.GEIGY AG) * Ansprüche * | 1,35,36 | |
| A | GB-A-1 325 061 (CIBA-GEIGY AG) * das ganze Dokument * | 1 | |
| P,X | EP-A-0 508 955 (CIBA-GEIGY AG) * Ansprüche * | 1-4,35, 36 | |

RECHERCHIERTE SACHGEBIETE (Int. Cl. 5): C07D

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 28 JULI 1993 | HENRY J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04E09)

EP 93 81 0382
-C-

UNVOLLSTÄNDIGE RECHERCHE

Vollständig recherchierte Patentansprüche: 1-37

Nicht recherchierter Patentanspruch: 38

BEMERKUNG: Obwohl Anspruch 35 sich auf ein Verfahren zur Behandlung des menschlichen Körpers bezieht (Art. 52(4) EPU) wurde die Recherche durchgeführt und gründete sich auf die angeführten Wirkungen der Verbindung

Die Ansprüche dürfen sich nicht auf Bezugnahmen auf die Beschreibung stützen (Regel 29(6) EPU)